# EUROPÄISCHE PATENTSCHRIFT

(11) **EP 0 863 999 B1**
(45) Veröffentlichungstag und Bekanntmachung des Hinweises auf die Patenterteilung: **28.03.2001**
(21) Anmeldenummer: 96939934.4
(22) Anmeldetag: 27.11.1996
(51) Int. Cl.: C12Q 1/68, C12Q 1/70, C12N 9/12

(54) **VERFAHREN ZUM NACHWEIS VON TELOMERASE-AKTIVITÄT**
METHOD OF DETECTING TELOMERASE ACTIVITY
PROCEDE POUR DETECTER L'ACTIVITE D'UNE TELOMERASE

(30) Priorität: 28.11.1995 DE 19544317; 24.10.1996 DE 19644302
(43) Veröffentlichungstag der Anmeldung: 16.09.1998
(73) Patentinhaber: Roche Diagnostics GmbH, 68305 Mannheim (DE)
(72) Erfinder: EMRICH, Thomas, D-82393 Iffeldorf (DE); LEYING, Hermann, D-83673 Bichl (DE); HINZPETER, Matthias, D-80689 München (DE); KARL, Gerlinde, D-82467 Garmisch-Partenkirchen (DE)
(74) Vertreter: Weiss, Wolfgang, Dipl.-Chem. Dr.
(86) Internationale Anmeldenummer: EP9605245
(87) Internationale Veröffentlichungsnummer: WO9720069

(56) Entgegenhaltungen:
- EP-A- 0 437 774
- WO-A-91/04753
- WO-A-93/09813
- WO-A-93/11261
- WO-A-95/13381
- SCIENCE, Bd. 266, - 23.Dezember 1994 Seiten 2011-2015, XP002028668 KIM N. W. ET AL.,: "Specific association of human telomerase activity with immortal cells and cancer" in der Anmeldung erwähnt
- NUCLEIC ACID RESEARCH, Bd. 23, Nr. 18, - 25.September 1995 Seiten 3794-3795, XP002028669 WRIGHT W. ET AL.,: "Modification of a telomeric repeat amplification protocol (TRAP) result in increased reliability, linearity and sensitivity"
- PROC. NATL. ACAD. SCI. USA, Bd. 91, - April 1994 Seiten 2900-2904, XP002028670 COUNTER C. M. ET AL.,: "Telomerase activity in human ovarian carcinoma" in der Anmeldung erwähnt
- CELL, Bd. 59, - 3.November 1989 Seiten 521-529, XP002028671 MORIN G. B.: "The human telomere terminal transferase enzyme is a ribonucleoprotein that synthesizes TTAGGG repeats" in der Anmeldung erwähnt

### SEQUENZPROTOKOLL

(1) ALLGEMEINE ANGABEN:
   (i) ANMELDER:
      (A) NAME: Boehringer Mannheim GmbH
      (B) STRASSE: Sandhofer Str. 116
      (C) ORT: Mannheim
      (E) LAND: Deutschland
      (F) POSTLEITZAHL: 68298
   (ii) BEZEICHNUNG DER ERFINDUNG: Telomerase-Detektion
   (iii) ANZAHL DER SEQUENZEN: 15
   (iv) COMPUTER-LESBARE FASSUNG:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.30 (EPA)
(2) ANGABEN ZU SEQ ID NO: 1:
   (i) SEQUENZKENNZEICHEN:
      (A) LÄNGE: 18 Basenpaare
      (B) ART: Nucleotid
      (C) STRANGFORM: Einzelstrang
      (D) TOPOLOGIE: linear
   (xi) SEQUENZBESCHREIBUNG: SEQ ID NO: 1:
   AATCCGTCGA GCAGAGTT 18
(2) ANGABEN ZU SEQ ID NO: 2:
   (i) SEQUENZKENNZEICHEN:
      (A) LÄNGE: 32 Basenpaare
      (B) ART: Nucleotid
      (C) STRANGFORM: Einzelstrang
      (D) TOPOLOGIE: linear
   (xi) SEQUENZBESCHREIBUNG: SEQ ID NO: 2:
   ACCCTTTTAG TCAGTGTGGA AAATCTCTAG CA 32
(2) ANGABEN ZU SEQ ID NO: 3:
   (i) SEQUENZKENNZEICHEN:
      (A) LÄNGE: 18 Basenpaare
      (B) ART: Nucleotid
      (C) STRANGFORM: Einzelstrang
      (D) TOPOLOGIE: linear
   (xi) SEQUENZBESCHREIBUNG: SEQ ID NO: 3:
   TTAGGGTTAG GGTTAGGG 18
(2) ANGABEN ZU SEQ ID NO: 4:
   (i) SEQUENZKENNZEICHEN:
      (A) LÄNGE: 24 Basenpaare
      (B) ART: Nucleotid
      (C) STRANGFORM: Einzelstrang
      (D) TOPOLOGIE: linear
   (xi) SEQUENZBESCHREIBUNG: SEQ ID NO: 4:
   CCCTTACCCT TACCCTTACC CTAA 24
(2) ANGABEN ZU SEQ ID NO: 5:
   (i) SEQUENZKENNZEICHEN:
      (A) LÄNGE: 24 Basenpaare
      (B) ART: Nucleotid
      (C) STRANGFORM: Einzelstrang
      (D) TOPOLOGIE: linear
   (xi) SEQUENZBESCHREIBUNG: SEQ ID NO: 5:
   GCGCGGCTAA CCCTAACCCT AACC 24
(2) ANGABEN ZU SEQ ID NO: 6:
   (i) SEQUENZKENNZEICHEN:
      (A) LÄNGE: 19 Basenpaare
      (B) ART: Nucleotid
      (C) STRANGFORM: Einzelstrang
      (D) TOPOLOGIE: linear
   (xi) SEQUENZBESCHREIBUNG: SEQ ID NO: 6:
   TTTTTTTTTT TTTTTTTMH 19
(2) ANGABEN ZU SEQ ID NO: 7:
   (i) SEQUENZKENNZEICHEN:
      (A) LÄNGE: 24 Basenpaare
      (B) ART: Nucleotid
      (C) STRANGFORM: Einzelstrang
      (D) TOPOLOGIE: linear
   (xi) SEQUENZBESCHREIBUNG: SEQ ID NO: 7:
   CCCTAACCCT AACCCTAACC CTAA 24
(2) ANGABEN ZU SEQ ID NO: 8:
   (i) SEQUENZKENNZEICHEN:
      (A) LÄNGE: 64 Basenpaare
      (B) ART: Nucleotid
      (C) STRANGFORM: Einzelstrang
      (D) TOPOLOGIE: linear
   (xi) SEQUENZBESCHREIBUNG: SEQ ID NO: 8:
   GATCCAATCC GTCGAGCAGA GTTAACTACC TTCAACTCCA TCATGAGGGT TAGGGTTAGG 60
   GATC 64
(2) ANGABEN ZU SEQ ID NO: 9:
   (i) SEQUENZKENNZEICHEN:
      (A) LÄNGE: 22 Basenpaare
      (B) ART: Nucleotid
      (C) STRANGFORM: Einzelstrang
      (D) TOPOLOGIE: linear
   (xi) SEQUENZBESCHREIBUNG: SEQ ID NO: 9:
   CATGATGGAG TTGAAGGTAG TT 22
(2) ANGABEN ZU SEQ ID NO: 10:
   (i) SEQUENZKENNZEICHEN:
      (A) LÄNGE: 21 Basenpaare
      (B) ART: Nucleotid
      (C) STRANGFORM: Einzelstrang
      (D) TOPOLOGIE: linear
   (xi) SEQUENZBESCHREIBUNG: SEQ ID NO: 10:
   AAGACGGGTG AGCTGGTGAT A 21
(2) ANGABEN ZU SEQ ID NO: 11:
   (i) SEQUENZKENNZEICHEN:
      (A) LÄNGE: 36 Basenpaare
      (B) ART: Nucleotid
      (C) STRANGFORM: Einzelstrang
      (D) TOPOLOGIE: linear
   (xi) SEQUENZBESCHREIBUNG: SEQ ID NO: 11:
   AATCCGTCGA GCAGAGTTCC CGCCTGATGA ATGCTC 36
(2) ANGABEN ZU SEQ ID NO: 12:
   (i) SEQUENZKENNZEICHEN:
      (A) LÄNGE: 44 Basenpaare
      (B) ART: Nucleotid
      (C) STRANGFORM: Einzelstrang
      (D) TOPOLOGIE: linear
   (xi) SEQUENZBESCHREIBUNG: SEQ ID NO: 12:
   GCGCGGCTAA CCCTAACCCT AACCAGAAAC TGCCGGAAAT CGTC 44
(2) ANGABEN ZU SEQ ID NO: 13:
   (i) SEQUENZKENNZEICHEN:
      (A) LÄNGE: 30 Basenpaare
      (B) ART: Nucleotid
      (C) STRANGFORM: Einzelstrang
      (D) TOPOLOGIE: linear
   (xi) SEQUENZBESCHREIBUNG: SEQ ID NO: 13:
   GGGCGGCCCT TACCCTTACC CTTACCCTAA 30
(2) ANGABEN ZU SEQ ID NO: 14:
   (i) SEQUENZKENNZEICHEN:
      (A) LÄNGE: 18 Basenpaare
      (B) ART: Nucleotid
      (C) STRANGFORM: Einzelstrang
      (D) TOPOLOGIE: linear
   (xi) SEQUENZBESCHREIBUNG: SEQ ID NO: 14:
   CCTAACCCTA ACTCTGCT                                                   18

   (2) ANGABEN ZU SEQ ID NO: 15:
   (i) SEQUENZKENNZEICHEN:
      (A) LÄNGE: 18 Basenpaare
      (B) ART: Nucleotid
      (C) STRANGFORM: Einzelstrang
      (D) TOPOLOGIE: linear
   (xi) SEQUENZBESCHREIBUNG: SEQ ID NO: 15:
   GGGCGGCCCT TACCCTTA                                                   18

## Beschreibung

Die vorliegende Erfindung betrifft ein Verfahren zum Nachweis von Telomerase-Aktivität sowie dafür geeignete Reagenzien.

Telomere sind spezifische Strukturen an den Enden von Chromosomen und bestehen bei eukaryontischen Organismen aus einer Anhäufung von sich wiederholenden definierten Nucleotidsequenzen (Repeats), die beispielsweise bei Menschen die Sequenz TTAGGG enthalten. In somatischen Zellen führt jede Replikation der Zelle unweigerlich zu einer Verkürzung der Telomerenden und ab einer bestimmten Unterschreitung der Telomerlänge schließlich zum Absterben der Zelle.

Virus-transformierte oder immortalisierte Zellen dagegen zeigen keine Reduzierung in ihrer Telomerlänge. Dies ist durch die Aktivität eines endogenen Ribonucleoproteins in diesen Zellen begründet, welches als Telomerase bezeichnet wird und in einer der Reversen Transkriptase ähnlichen Reaktion der Telomerverkürzung entgegenwirken kann.

Da die Expression der Telomerase nach bisherigen Erkenntnissen auf Tumorzellen, Keimzellen und immortalisierte Zellen beschränkt ist, stellt dieses Protein einen vielversprechenden Parameter für eine Diagnostik von Tumoren sowie einen Angriffspunkt für eine Tumortherapie dar (vgl. z.B. die Übersichtsartikel von Greider, 1994, Curr. Oppin. Gen. Dev. 4, 203-211; Counter et al., 1994, Proc. Natl. Acad. Sci. USA 91, 2900-2904 und Hiyama et al., 1995, Nature Med. 1, 249-255).

Die in der Literatur beschriebenen Verfahren zum Nachweis von Telomerase-Aktivität basieren alle auf einer in vitro-Detektion der Enzymaktivität. Ein immunologischer Nachweis des humanen Enzyms ist derzeit nicht möglich, da seine Proteinsequenz noch nicht bekannt ist. Lediglich die Sequenz der Telomerase aus Tetrahymena wurde kürzlich beschrieben (Collins et al., 1995, Cell 81, 677-686).
Bei den in der Literatur beschriebenen Nachweisverfahren unterscheidet man zwischen zwei prinzipiellen Methoden. Die erste Methode beruht darauf, daß ein aus der Telomersequenz abgeleitetes synthetisches Oligonucleotid als Primer dient. Dieser Primer wird zusammen mit unmarkierten Didesoxynucleotiden und einem radioaktiv markierten Desoxynucleotid einer Probe, z.B. einem Telomerase enthaltenden Zellextrakt zugesetzt, wobei der Primer durch die Telomerase spezifisch elongiert und das Syntheseprodukt dabei radioaktiv markiert wird. Der Reaktionsansatz wird anschließend gelelektrophoretisch aufgetrennt und das Bandenmuster durch Exposition eines Röntgenfilms mit nachfolgender Entwicklung sichtbar gemacht (Morin, 1989, Cell 59, 521-529; Nilsson et al., 1993, Oncogene 9, 3043-3048).

Auch bei der anderen Nachweismethode wird zunächst ein Telomerase-spezifisches Elongationsprodukt erzeugt. Dieses wird jedoch in einer anschließenden Polymerasekettenreaktion (PCR) amplifiziert und gleichzeitig durch Zusatz von radioaktiven Desoxynucleotiden markiert. Der Nachweis der markierten PCR-Produkte erfolgt durch Gelelektrophorese (Kim et al. 1994, Science 266, 2011-2015).

WO 95/13381 beschreibt ein Verfahren zum Nachweis von Telomerase-Aktivität, wobei ein zu testender Zellextrakt mit einem Primer in Kontakt gebracht wird, der keine telomeren Repeatsequenzen enthält, wobei die Telomerase eine Extension des Primers durch Anfügen telomerer Repeatsequenzen katalysieren kann. Anschließend erfolgt eine Amplifikationsschritt unter Zugabe eines zweiten Primers. Der Nachweis der Telomerase-Aktivität erfolgt schließlich durch gelelektrophoretische Auftrennung der resultierenden Amplifikationsprodukte.

Diese Nachweisverfahren des Standes der Technik besitzen jedoch einige Nachteile. So hat ein Nachweisverfahren ohne Amplifikationsschritt eine zu geringe Sensitivität für routinemäßige Anwendungen, da Extraktmengen mit 10⁶ bis 10⁷ Zellen eingesetzt werden müssen. Zur Untersuchung von primärem Tumormaterial, das nur in geringer Menge zur Verfügung steht, ist diese Methode deshalb nicht verwendbar. Weiterhin bewegt sich die Expositionsdauer der Gele im Bereich von 2 bis 7 Tagen, was ebenfalls auf die geringe Sensitivität zurückzuführen ist. Bei der einen Amplifikationsschritt enthaltenden Nachweismethode tritt dieser Nachteil zwar nicht auf, da routinemäßig nur 10⁵ Zelläquivalente pro Test eingesetzt werden müssen und 10³ Zelläquivalente reproduzierbar nachgewiesen werden können. Die Expositionszeiten der Gele betragen jedoch auch bei diesen Verfahren noch mindestens einen Tag (Kim et al., 1994, Supra; Chadeneau et al., 1995, Cancer Res. 55, 2533-2536).

Beide in der Literatur beschriebenen Nachweismethoden haben den Nachteil, daß die Markierung des Elongationsprodukts bzw. des PCR-Produkts mit radioaktiven Markierungsgruppen erfolgen muß, um befriedigende Ergebnisse zu erhalten. Dies führt zu hohen Expositionszeiten und zum unerwünschten Entstehen von radioaktivem Abfall. Weiterhin hat die elektrophoretische Auftrennung des Reaktionsansatzes, gefolgt von anschließender Exposition und Entwicklung eines Röntgenfilms einen hohen manuellen Aufwand zur Folge.

Darüberhinaus erlaubt keine der genannten Methoden einen hohen Probendurchsatz. Für eine Automatisierung, wie sie z.B. für die Routineanalytik oder für ein Effektor-Screening notwendig ist, sind sie nicht geeignet.

Die der vorliegenden Erfindung zugrundeliegende Aufgabe bestand somit darin, Nachteile der Verfahren des Standes der Technik mindestens teilweise zu beseitigen. Diese Aufgabe wird gelöst durch ein Verfahren zum Nachweis von Telomerase-Aktivität,
dadurch gekennzeichnet, daß man
(a) eine zu testende Probe bereitstellt,
(b) einen als Telomerasesubstrat geeigneten ersten Primer und Nucleosidtriphosphate zugibt und das Reaktionsgemisch unter Bedingungen inkubiert, bei denen eine Primerextension durch die Telomerase stattfinden kann,
(c) eine Amplifikation des durch die Telomerase erzeugten Extensionsprodukts durchgeführt,
(d) das in Schritt (c) erzeugte Amplifikationsprodukt an einer Festphase immobilisiert und
(e) das immobilisierte Amplifikationsprodukt qualitativ oder/und quantitativ nachweist.

Überraschenderweise wurde festgestellt, daß durch Immobilisierung des Amplifikationsprodukts die Spezifität der Telomerasereaktion beibehalten wird, d.h. ein positives Signal kann reproduzierbar auf eine Telomerase-Aktivität zurückgeführt werden. Die nach dem Stand der Technik erforderliche gelelektrophoretische Auftrennung des Reaktionsansatzes ist somit überflüssig. Weiterhin wird eine sehr hohe Sensitivität erreicht. In bestimmten Testformaten kann durch das erfindungsgemäße Verfahren sogar ein Sensitivitätsgewinn gegenüber den Verfahren des Standes der Technik erzielt werden. Außerdem ist bei dem erfindungsgemäßen Verfahren die Verwendung von nicht-radioaktiven Markierungen möglich und bevorzugt, wodurch die beim Umgang mit radioaktiven Substanzen auftretenden Schwierigkeiten vermieden werden können. Diese Vorteile machen das erfindungsgemäße Verfahren hervorragend für Routineanwendungen in automatisierten Nachweisgeräten geeignet.

Das erfindungsgemäße Verfahren ermöglicht einen spezifischen Nachweis von amplifizierten Telomerase-Extensionsprodukten, ohne daß eine Separation der Reaktionsprodukte erforderlich ist. Dies konnte nicht ohne weiteres erwartet werden, da sämtliche Amplifikationsansätze neben den Telomerase-Extensionsprodukten auch unspezifische Nebenprodukte wie z.B. Primer-Dimere, Repeatsequenzen enthalten. So mußte man befürchten, daß auch diese unspezifischen Produkte beim Nachweis mit einer gegen die Repeatsequenz gerichteten Fang- oder Detektionssonde erfaßt werden. Ebenso war zu befürchten, daß bei Zusatz eines internen Standards dieser stets mitdetektiert werden würde. Überraschenderweise wurde jedoch gefunden, daß durch Auswahl geeigneter Hybridisierungsbedingungen und gegebenenfalls durch Zusatz unmarkierter Oligonucleotide, die komplementär zu den Primersequenzen sind, ein hochspezifischer Nachweis von Telomeraseextensionsprodukten ohne Separationsschritt gelingt.

Der Nachweis der Telomerase-Aktivität erfolgt vorzugsweise durch Verwendung von Markierungsgruppen, insbesondere von nicht-radioaktiven Markierungsgruppen. Als nicht-radioaktive Markierungsgruppen können alle bekannten Markierungsgruppen verwendet werden, z.B. immunologisch reaktive Gruppen, z.B. Nucleotidanaloga oder Haptene, die mit einem Nachweisantikörper reagieren können, Enzyme wie etwa Peroxidase, Galactosidase oder alkalische Phosphatase, fluoreszierende oder lumineszierende Gruppen, z.B. Elektrochemilumineszenz-Gruppen, oder andere Nachweisgruppen wie etwa NMR-aktive Markierungsgruppen oder elektronendichte Gruppen. Bevorzugt sind immunologisch reaktive Gruppen wie etwa Nucleotidanaloga, z.B. Halogenderivatisierte Nucleotide wie etwa Br-dUTP, oder mit organischen Resten, die mindestens ein C-Atom enthalten, derivatisierte Nucleotide wie etwa CH₃-dCTP, oder Haptene, z.B. Digoxigenin, Digoxin, Fluorescein etc., lumineszierende Gruppen wie etwa lumineszierende Metallkomplexe, z.B. Ruthenium-Komplexe, und fluoreszierende Gruppen wie etwa Fluorescein.

Die nicht-radioaktiven Markierungsgruppen können einerseits direkt in das Amplifikationsprodukt eingebaut werden. Dieser Einbau kann beispielsweise durch Verwendung von markierten Nucleotiden oder/und markierten Primern erfolgen. Andererseits können die Amplifikationsprodukte auch indirekt z.B. durch Verwendung einer geeigneten Markierungssonde markiert werden, d.h. einer Sonde, die selbst eine oder mehrere Markierungsgruppen enthält und spezifisch mit dem Amplifikationsprodukt hybridisiert. Als Markierungssonden kann man z.B. Oligonucleotide oder/und Nucleinsäureanaloga verwenden. Die Hybridisierung der Sonde an das Amplifikationsprodukt kann vor oder/und nach dem Immobilisierungsschritt erfolgen.

Schritt (a) des erfindungsgemäßen Verfahrens umfaßt das Bereitstellen einer zu testenden Probe. Vorzugsweise ist diese Probe ein Zellextrakt, insbesondere ein Extrakt aus humanen Zellen. Der Zellextrakt kann jedoch auch aus anderen eukaryontischen Organismen wie etwa Hefen oder Tetrahymena stammen. Vorzugsweise wird der Zellextrakt durch Lyse von Zellen in einem Puffer hergestellt, der 0,01 - 5 Gew.-% eines nichtionischen oder/und zwitterionischen Detergens enthält. Andererseits ist eine Lyse der Probe auch durch gegebenenfalls mehrmaliges Auftauen/Einfrieren möglich. Anschließend werden vorzugsweise unlösliche Bestandteile, wie etwa zelluläre Rückstände, durch Zentrifugation oder/und Filtration entfernt und der Überstand gesammelt. Gute Ergebnisse werden mit einer Extraktmenge erhalten, die 10² bis 10⁵ Zelläquivalenten entspricht. Auch bei Verwendung von nur 1 - 10 Zelläquivalenten werden noch spezifische Signale erhalten. Wenn es sich bei der zu testenden Probe um Gewebe, z.B. Tumorgewebe, handelt, werden vorzugsweise 10 - 1000 ng Gewebe für einen Test verwendet.

Schritt (b) des erfindungsgemäßen Verfahrens umfaßt die Zugabe eines als Telomerasesubstrat geeigneten einzelsträngigen Primers zur Probe und die Inkubation des resultierenden Reaktionsgemisches unter Bedingungen, bei denen eine Primerextension durch die Telomerase stattfinden kann. Der Primer ist vorzugsweise ein Oligodesoxyribonucleotid. Die Länge des Primers ist vorzugsweise 10 - 50 und besonders bevorzugt 12 - 30 Nucleotide. Bei diesem Verfahren kann man einerseits einen ersten Primer verwenden, der frei von telomeren Repeatsequenzen ist. Ein bevorzugtes Beispiel für einen derartigen Primer ist ein von Morin et al. (1991), Nature 353, 454 - 456, beschriebener Primer P1 mit der in SEQ ID NO. 1 gezeigten Nucleotidsequenz.

Aus WO 91/04753 war die Verwendung von Konjugaten bekannt, die aus Antisense-Oligonukleotiden, die an ein an Liganden bindendes Molekül gekoppelt sind, bestehen. Das Ligandenbindende Molekül erkennt z.B. Zelloberflächenmoleküle, die für die Behandlung von Krankheiten, die durch Viren verursacht werden, nützlich sind. Als Antisense-Oligonukleotide werden in WO 91/04753 auch solche beschrieben, die zu retroviralen Genomsequenzabschnitten komplementär sind.

Es wurde nun weiterhin überraschenderweise festgestellt, daß für das erfindungsgemäße Verfahren auch Primer geeignet sind, die aus dem 5'-Bereich einer retroviralen LTR-Sequenz, z.B. aus dem 5'-Bereich der LTR-Sequenz von HIV stammen. Ein Beispiel für einen solchen Primer ist in SEQ ID NO. 2 gezeigt. Neben einzelsträngigen Primern können im übrigen auch doppelsträngige Primer mit einem 3'-Überhang eingesetzt werden.

Andererseits kann man auch einen ersten Primer verwenden, der telomere Repeatsequenzen enthält, z.B. den Primer P1-Telo mit der in SEQ ID NO. 3 gezeigten Nucleotidsequenz.

Bei Verwendung von nicht-radioaktiven Markierungsgruppen wird der Extensionschritt (b) vorzugsweise so durchgeführt, daß nur unmarkierte Nucleosidtriphosphate an den ersten Primer angefügt werden können. Der Grund hierfür ist, daß bei Anwesenheit von nicht-radioaktiv markierten Nucleosidtriphosphaten im Reaktionsgemisch, die von der Telomerase als Substrat verwendet werden können, eine partielle Hemmung der Telomerase-Aktivität gefunden wird, die zu einer etwas ineffizienteren Primerelongation führt. Dennoch ist in bestimmten Ausführungsformen der Erfindung der Einbau von nicht-radioaktiven Markierungsgruppen auch während der Primerextension möglich.

Vorzugsweise werden jedoch nicht-radioaktive Markierungsgruppen erst beim nachfolgenden Amplifikationsschritt (c) in das Produkt eingeführt. Dies kann beispielsweise dadurch erreicht werden, daß man nicht-radioaktiv markiertes CTP (kein Bestandteil der telomeren Repeat-Sequenzen) verwendet. In diesem Fall kann die Reaktion als "Eintopfreaktion" ohne Kompartimentierung durchgeführt werden. Andererseits können die nicht-radioaktiv markierten Nucleosidtriphosphate erst später mit dem Reaktionsgemisch in Kontakt gebracht werden. Dies kann beispielsweise durch Kompartimentierung erreicht werden, wobei die nicht-radioaktiv markierten Nucleosidtriphosphate während des Extensionsschritts (b) vom Reaktionsgemisch durch eine entfernbare Barriere, z.B. eine bei höheren Temperaturen schmelzbare Wachsschicht abgetrennt sind. Andererseits ist natürlich auch eine sequentielle Zugabe der Reaktanten möglich.

Weiterhin kann es bevorzugt sein, daß man nach dem Extensionsschritt (b) eine zusätzliche Template-unabhängige Elongation des von der Telomerase erzeugten Extensionsprodukts durchführt. Diese Elongation wird vorzugsweise durch eine enzymatische Reaktion, z.B. durch Anfügen von Nucleotiden, z.B. durch eine Polyadenylierung mittels Terminaler Transferase oder durch Anligation von kurzen DNA-Fragmenten mittels DNA-Ligase durchgeführt.

Schritt (c) des erfindungsgemäßen Verfahrens umfaßt eine Amplifikation des durch die Telomerase erzeugten Extensionsprodukts. Die Art des Amplifikationsschritts ist für das erfindungsgemäße Verfahren nicht kritisch. Typischerweise erfolgt die Amplifikation durch Zugabe eines geeigneten Enzyms, das eine Polymerisierung von Nucleinsäuren bewirken kann, z.B. einer Nucleinsäure-Polymerase oder einer Nucleinsäure-Ligase. Vorzugsweise verwendet man ein thermostabiles Enzym und führt die Amplifikation in mehreren Zyklen durch.

Vorzugsweise werden zur Amplifikation zwei Primer verwendet, wobei als erster Primer das Telomerase-Substrat und als zweiter Primer ein geeigneter komplementärer Primer verwendet werden kann. Durch enzymatische Katalyse z.B. durch eine Template-abhängige DNA-Polymerase entsteht das Amplifikationsprodukt durch Anfügen von Nucleotiden an den ersten und zweiten Primer. Der zweite Primer kann vorzugsweise mit der telomeren Repeatsequenz hybridisieren wie etwa der in SEQ ID NO. 4 gezeigte Primer P2. Besonders bevorzugt wird hierzu ein sogenannter Ankerprimer verwendet, der an seinem 5'-Ende einen nicht zur telomeren Repeatsequenz komplementären Bereich enthält. Ein derartiger Ankerprimer hat die Vorteile, daß keine Amplifikationsprodukte entstehen können, die länger als das ursprüngliche Template sind, und daß Primer-Dimere, die bei Verwendung eines Repeatsequenzen enthaltenden Primers ebenfalls Repeatsequenzen enthalten müssen, nicht verlängert werden. Besonders bevorzugt werden Ankerprimer verwendet, die an ihrem 5'-Ende eine von Repeatsequenzen freie Extension enthalten, die mindestens 4 und insbesondere mindestens 5 nt lang ist. Günstigerweise ist die Länge dieses Sequenzbereichs 4 - 20 Nucleotide. Am meisten bevorzugt sind Ankerprimer mit einer GC-reichen Sequenz am 5'-Ende. Oligonucleotide oder Nucleinsäureanaloge, vorzugsweise mit einer Länge von 10 - 50 nt, die eine zu einer telomeren Repeatsequenz komplementäre Sequenz und 5'-seitig davon von Repeatsequenz freie Extension aufweisen, sind generell als Primer in einem Verfahren zum Nachweis von Telomerase-Aktivität geeignet. Ein Beispiel für einen geeigneten Ankerprimer mit einer 5'-seitigen 6 nt langen Ankersequenz ist der in SEQ ID NO. 5 gezeigte Primer TE-ACT. Ein besonders bevorzugter Ankerprimer ist der in SEQ ID NO. 13 dargestellte Primer TE-3.2. Mit diesem Primer und mit analogen Primern, denen im Vergleich zu TE-3.2 am 3'-Ende bis maximal 3 Nucleotide fehlen, werden besonders gute Testresultate erhalten.

Das Enzym ist vorzugsweise eine thermostabile DNA-Polymerase, mit der viele Amplifikationszyklen ohne Inaktivierung der Polymerase durchgeführt werden können. Ein besonders bevorzugtes Amplifikationsverfahren ist die Methode der Polymerase-Ketten-Reaktion (PCR).

Wenn nach der Extension eine zusätzliche Elongation, z.B. durch Terminale Transferase erfolgt, kann für den anschließenden Amplifikationschritt (c) ein zweiter Primer verwendet werden, der komplementär zu dem durch Elongation an das Extensionsprodukt angefügten Sequenzabschnitt ist. Ein Beispiel hierfür ist der in SEQ ID NO. 6 gezeigte Primer P3.

Alternativ kann die Amplifikation durch andere, dem Fachmann bekannte Methoden erfolgen. So kann die Reaktion auch durch eine Template-abhängige DNA-Ligase katalysiert werden, wobei das Amplifikationsprodukt durch Anfügen eines Oligodeoxyribonucleotids an die Primer durch die DNA-Ligase erzeugt wird. Vorzugsweise ist die DNA-Ligase eine thermostabile DNA-Ligase und besonders bevorzugt wird ein derartiges Verfahren nach der Technik der Ligase-Ketten-Reaktion (LCR) durchgeführt.

Schritt (d) des erfindungsgemäßen Verfahrens umfaßt die Immobilisierung des Amplifikationsprodukts an einer Festphase. Als Festphase kann beispielsweise die Wand des Reaktionsgefäßes dienen. Alternativ können auch partikuläre Festphasen eingesetzt werden. Vorzugsweise wird die Festphase ausgewählt aus Mikrotiterplatten, Mikroreaktionsgefäßen, Membranen, Mikrochips, Biocore-Systemen und gegebenenfalls magnetischen Mikrobeads. Aufgrund des Immobilisierungsschritts wird im Vergleich zu den Verfahren des Standes der Technik eine große Zeitersparnis bei geringerem manuellen Aufwand erreicht. Weiterhin ist ein hoher Probenduchsatz und eine Automatisierung, z.B. für die Routineanalytik oder für das Screening von Effektoren möglich.

Die Immobilisierung der Amplifikationsprodukte an der Festphase kann grundsätzlich nach jeder bekannten Methode erfolgen, z. B. durch adsorptive Bindung. Vorzugsweise erfolgt die Immobilisierung jedoch durch spezifische Wechselwirkungen, z.B. über Ankergruppen. Beispiele für geeignete Ankergruppen sind immunologisch reaktive Gruppen, die mit einem Festphasegebundenen Antikörper reagieren können, oder andere Gruppen, die zu einer hochaffinen Bindung mit einem immobilisierten Partner in der Lage sind. Ein bevorzugtes Beispiel für eine Ankergruppe ist Biotin, das mit hoher Affinität an eine mit Avidin oder Streptavidin beschichtete Festphase binden kann. Für hohe Probenzahlen kan beispielsweise die von Savoysky et al. (Nucleic Acids Res. 24 (1996), 1175-1176) beschriebene Immobilisierungsmethode verwendet werden, bei der ein biotinylierter Telomerase-Extensionsprimer verwendet wird, so daß bei der Amplifikationsreaktion Produkte entstehen, die Biotin als Immobilisierungsgruppe aufweisen. Gleichzeitig wird während der Amplifikation [Me-³H] TTP eingebaut. Die biotinylierten und [³H]-markierten Amplifikationsprodukte werden an Streptavidinbeschichtete Fluoromikropartikel immobilisiert. Diese Immobilisierung wird aufgrund von Wechselwirkungen der von [³H] freigesetzten β-Quanten mit den Fluoromikropartikeln durch Szintillationsmessung nachgewiesen.

Die Einführung der Ankergruppen in das Amplifikationsprodukt kann in verschiedenen Stadien des erfindungsgemäßen Verfahrens erfolgen. So kann man beispielsweise einen oder mehrere Primer verwenden, welche bereits Ankergruppen, z.B. Biotingruppen, enthalten. Andererseits können biotinylierte Nucleotide auch durch Elongation des primären Extensionsprodukts, z.B. mit Terminaler Transferase oder mit DNA-Ligase, oder beim Amplifikationsschritt (c) eingeführt werden.

In einer bevorzugten Ausführungsform der vorliegenden Erfindung ist es jedoch gar nicht erforderlich, daß das Amplifikationsprodukt selbst eine Ankergruppe enthält. Eine Verankerung an die Festphase kann beispielsweise auch durch Zugabe einer Fangsonde erreicht werden, die eine oder mehrere Ankergruppen trägt und mit dem Amplifikationsprodukt eine unter den Reaktionsbedingungen stabile Hybridisierung eingehen kann. Beispiele für geeignete Fangsonden sind Oligonucleotide, welche telomere Repeatsequenzen oder dazu komplementäre Sequenzen enthalten und eine oder mehrere Ankergruppen, z.B. Biotingruppen enthalten. Besonders bevorzugt sind Fangsonden, die an ihrem 5'-Ende eine Ankergruppe enthalten. Als Fangsonde kann z.B. das in SEQ ID NO. 7 gezeigte Oligonucleotid P4 mit einer 5'-Biotingruppe eingesetzt werden.

Andererseits können auch Nucleinsäureanaloga als Fangsonden eingesetzt werden, z.B. peptidische Nucleinsäuren (Nielsen et al. (1991), Science 254, 1497 - 1500 und Dueholm et al. (1994), J.Org.Chem. 59, 5767 - 5773). Peptidische Nucleinsäuren weisen ein über Säureamidbindungen verknüpftes Rückgrat auf, das Nucleobasen als Seitengruppen enthält. Die Verwendung von peptidischen Nucleinsäuren als Fangsonden - und auch als Markierungssonden - ist in bestimmten Ausführungsformen des erfindungsgemäßen Verfahrens bevorzugt.

Schritt (e) des erfindungsgemäßen Verfahrens umfaßt den qualitativen oder/und quantitativen Nachweis des Amplifikations-produkts. Der Nachweis erfolgt über die im Amplifikationsprodukt enthaltenen Markierungsgruppen bzw. über die an das Amplifikationsprodukt gebundenen Markierungssonden auf an sich bekannte Weise. Vorzugsweise erfolgt der Nachweis bei Verwendung von nicht-radioaktiven Markierungsgruppen in automatisierten Meßvorrichtungen. Bevorzugt sind Meßvorrichtungen, in denen der Nachweis der Markierungsgruppen durch colorimetrische oder/und spektrophotometrische Methoden erfolgt, z.B. durch enzymatische Umsetzung eines Substrats bzw. durch Chemilumineszenz oder Fluoreszenz.

Der Nachweis wird vorzugsweise unter solchen Bedingungen durchgeführt, die eine spezifische Detektion von amplifizierten Telomeraseextensionsprodukten auch in Anwesenheit unspezifischer Nebenprodukte ermöglichen. Hierzu kann man Hybridisierungsbedingungen auswählen, die eine solche spezifische Detektion erlauben, oder/und dem Ansatz unmarkierte Oligonucleotide oder Nucleinsäureanaloga (Kompetitoren) zusetzen, die komplementär zu den Primersequenzen sind, so daß diese Sequenzbereiche maskiert werden und die Hybridisierung mit der Fangoder Detektionssonde spezifisch an internen Sequenzen der Amplifikationsprodukte erfolgt.

Beispiele für geeignete Hybridisierungsbedingungen sind 37°C und ein Formamid-freier oder Formamid-enthaltender Puffer (z.B. 5xSSC, 10 % Formamid, 0,1 % Sarkosyl, 0,02 % SDS, 1 % Blockierungsreagenz in Maleinsäurepuffer). Beispiele für geeignete unmarkierte Kompetitoren sind die in SEQ ID NO. 14 und 15 angegebenen Oligonucleotide.

Im folgenden sind einige bevorzugte Ausführungsformen für die Testdurchführung angegeben:

### Version 1:

Als Telomerasesubstrat wird ein von telomer-spezifischen Repeats freier Primer verwendet, z.B. mit der in SEQ ID NO. 1 oder 2 dargestellten Sequenz. Alternativ kann auch ein Repeatenthaltender Primer, z.B. P1-Telo (SEQ ID NO. 3) verwendet werden. Dieser Primer wird zusammen mit unmarkierten Nucleosidtriphosphaten dATP,dGTP und dTTP im Extensionsschritt (b) eingesetzt. Das durch die Telomerase erzeugte Extensionsprodukt wird dann in Schritt (c) einer Amplifikation unterzogen. Die für die Amplifikation benötigten Komponenten können, sofern sie die Extensionsreaktion nicht stören, bereits in Schritt (b) im Reaktionsgemisch vorliegen. Andere Komponenten liegen in kompartimentierter Form, z.B. unter einer schmelzbaren Wachsschicht, im gleichen Reaktionsgefäß vor.

Das Reaktionsgemisch in Schritt (c) enthält neben den bereits in Schritt (b) anwesenden Komponenten einen zweiten Primer, der eine Sequenz komplementär zur humanen Telomer-Repeat-Sequenz enthält, z.B. den Primer P2 mit der in SEQ ID NO. 4 gezeigten Sequenz, oder den Primer TE-ACT mit der in SEQ ID NO.5 gezeigten Sequenz, mindestens ein mit einer Ankergruppe versehenes Nucleosid-triphosphat, z.B. biotinyliertes dUTP, mindestens ein nicht-radioaktiv markiertes Nucleosidtriphosphat, z.B. Br-dUTP, Br-dCTP, CH₃-dCTP, DIG-dUTP oder DIG-dCTP, und eine thermostabile DNA-Polymerase, z.B. Taq-Polymerase.

Die Immobilisierung des Amplifikationsprodukts gemäß Schritt (d) erfolgt auf einer Mikrotiterplatte, die mit einem Konjugat aus Streptavidin und Rinderserumalbumin beschichtet ist. Der Nachweis des Amplifikationsprodukts erfolgt anschließend mit Hilfe eines Konjugats aus einem gegen die Markierungsgruppe, z.B. Br-dU, Br-dC, CH₃-dC bzw. DIG gerichteten Antikörper oder Antikörperfragments und einem Enzym, z.B. Peroxidase.

### Version 2:

Alternativ zu Version 1 wird das in Schritt (b) erzeugte Telomerase-Extensionsprodukt mit Hilfe von Terminaler Transferase und einem oder mehreren Nucleotiden, z.B. dATP, elongiert. Die Amplifikation erfolgt in diesem Fall durch Zusatz eines Oligo-dT-Primers, z.B. des Primers P3 mit der in SEQ ID NO. 6 gezeigten Sequenz, sowie markierten und unmarkierten Nucleotiden (siehe Version 1). Der Nachweis des Amplifikationsprodukts erfolgt wie oben beschrieben.

Diese Version bietet Sensitivitätsvorteile, da eine zusätzliche Einführung von Markierungsgruppen (ca. 50 - 100 Markierungsgruppen pro Amplifikationsprodukt) erfolgen kann. Dies ist insbesondere bei kurzen Telomer-Produkten von Bedeutung. Zusätzlich werden bei dieser Versuchsdurchführung ausschließlich Telomerase-katalysierte Vollänge-Extensionsprodukte amplifiziert, da durch die Wahl des Primers eine Hybridisierung mit Repeatsequenzen innerhalb des Elongationsprodukts ausgeschlossen wird. Dies führt ebenfalls zu einer erhöhten Dichte an Markierungsgruppen.

Alternativ kann die Immobilisierung der Amplifikationsprodukte bei den Versionen 1 und 2 durch Einsatz eines biotinylierten ersten oder/und zweiten Primers anstelle des Einbaus von biotinyliertem Nucleosidtriphosphat erfolgen. Die Biotinylierung der Primer kann beispielsweise an ihrem 5'-Ende erfolgen.

### Version 3:

Im Gegensatz zu Version 1 und 2 wird ein Amplifikationsprodukt erzeugt, das nur Markierungsgruppen, aber keine Festphasenankergruppen enthält. Bei dieser Version wird daher während der PCR nur eine einfache Markierung der DNA durch Zusatz von nicht-radioaktiv markierten Nucleotiden durchgeführt. Die Immobilisierung erfolgt durch Denaturierung der Amplifikationsprodukte mit anschließende Hybridisierung an biotinylierte Fangsonden, z.B. ein Oligonucleotid mit der in SEQ ID NO. 7 gezeigten Nucleotidsequenz.

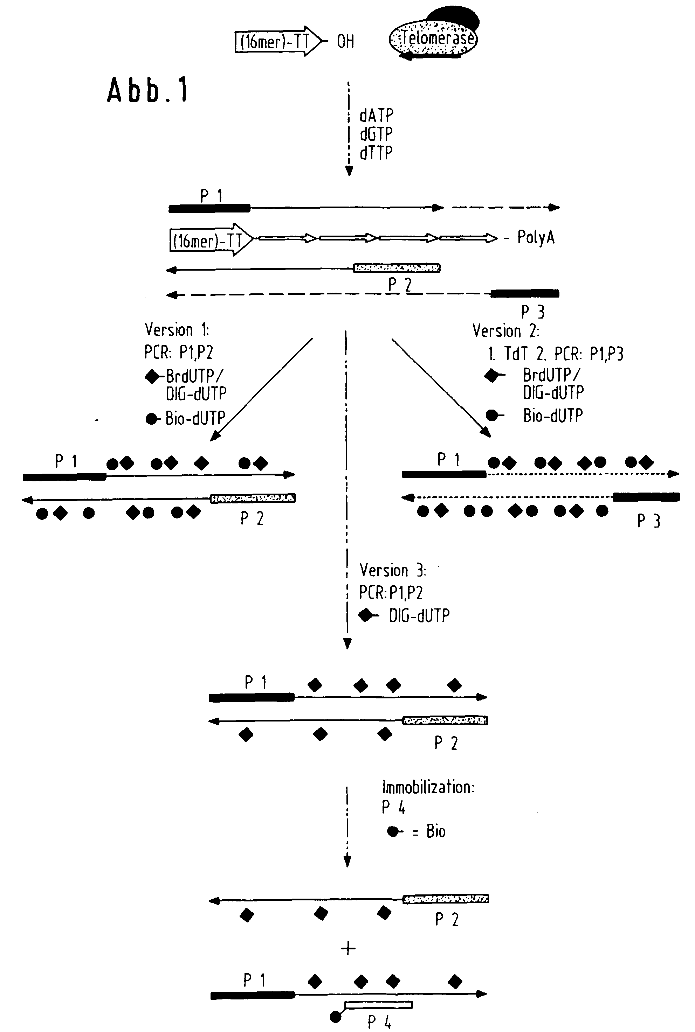

Bei allen Varianten ist alternativ eine sequentielle Testführung ohne Kompartimentierung durch Wachs möglich. Die Gesamtdauer aller Testdurchführungen beträgt maximal ca. 10 h und vorzugsweise maximal 6 bis 8 h. Eine schematische Darstellung der Versionen 1 - 3 des erfindungsgemäßen Verfahrens ist in Abb. 1 gezeigt.

### Version 4:

Die Telomerase-katalysierte Extension des Telomerase-Substrats, z.B. des Primers P1 bzw. eines biotinylierten Primers P1, erfolgt analog Version 1. Anschließend erfolgt eine Amplifikation des Extensionsprodukts unter Zusatz eines zweiten Primers, z.B. des Primers TE-ACT. Die Amplifikation findet in Abwesenheit von markierten Nucleotiden statt. Daher ist während Schritt (b) keine Kompartimentierung notwendig. Nach der Amplifikation werden analog Version 3 die Amplifikationsprodukte denaturiert und (i) bei Verwendung eines biotinylierten Primers mit einer Markierungssonde, z.B. einem DIG-Gruppen-enthaltendem Oligonucleotid, oder (ii) bei Verwendung von nicht-biotinylierten Primern mit einer Markierungssonde und einer biotinylierten Fangsonde hybridisiert. Der Nachweis erfolgt wie in Version 1 beschrieben.

Zusätzlich kann in einer bevorzugten Ausführungsform des erfindungsgemäßen Verfahrens eine Standardisierung der Amplifikationsreaktion durch Zugabe einer vorbestimmten Menge eines Amplifikationsstandards erfolgen.

Ein derartiger Amplifikationsstandard kann beispielsweise hergestellt werden, indem man nach Klonierung eines Telomerase-Extensionsprodukts in einen Klonierungsvektor 3'-seitig der dem ersten Primer entsprechenden Sequenz eine beliebige, nicht in den Telomerase-Extensionsprodukten enthaltene DNA-Sequenz mit Hilfe rekombinanter Techniken einfügt. Eine definierte Menge dieses Konstrukts wird dann während der Amplifikationsreaktion dem Ansatz zugesetzt und zusammen mit den Telomerase-Extensionsprodukten mit Hilfe der ersten und zweiten Primer amplifiziert. Ein Aliquot des Amplifikationsansatzes wird dann mit Hilfe einer ersten Fangsonde und ein weiteres Aliquot mit Hilfe einer zweiten Fangsonde analysiert. Mit der ersten Fangsonde lassen sich Amplifikationsprodukte nachweisen, die sowohl von Telomerase-Elongationsprodukten als vom zugesetzten Standard resultieren, während die zweite Fangsonde lediglich einen Nachweis der vom Standard abgeleiteten Amplifikationsprodukte ermöglicht. Eine Normierung der Meßwerte auf den Standardwert erlaubt nun eine quantitative Aussage über die Telomerase-Aktivität in der getesteten Probe.

Bei Verwendung von 2 oder mehreren unterschiedlichen Markierungsgruppen, die so ausgewählt werden, daß sie nebeneinander nachweisbar sind, können mehrere Detektionen, z.B. die Detektion der Telomerase-Extensionsprodukte und des Standards, sequentiell im gleichen Reaktionsgefäß erfolgen, d.h. die Untersuchung von zwei separaten Aliquots entfällt.

Ein Vorteil des erfindungsgemäßen Verfahrens besteht weiterhin darin, daß Standards eingesetzt werden können, die beim Nachweis der Telomeraseextensionsprodukte, z.B. durch Immunoblot nach Gelelektrophorese, eine Bande ergeben, die außerhalb der Banden der Telomeraseextensionsprodukte liegt. Somit ist eine bessere Quantifizierung möglich.

Alternativ lassen sich bei geeigneter Auswahl der Fangsonden mit der ersten Fangsonde lediglich die vom Standard resultierenden Amplifikationsprodukte und mit einer zweiten Fangsonde lediglich die vom Telomerase-Extensionsprodukt stammenden Amplifikationsprodukte immobilisieren.

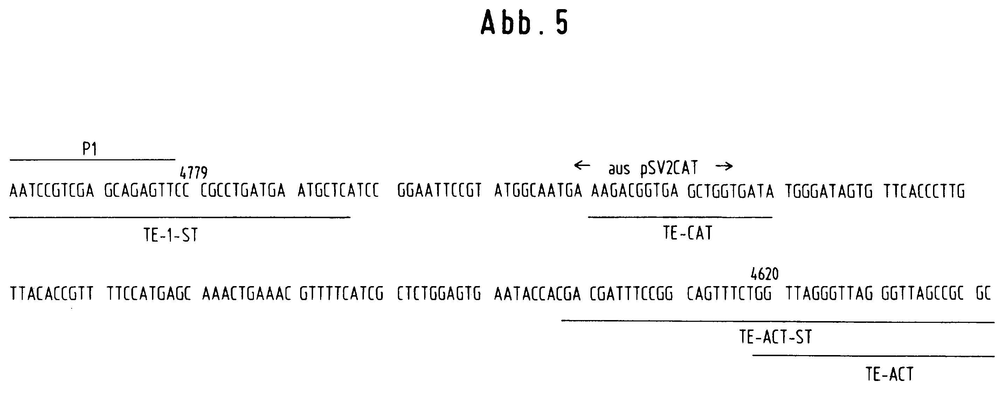

Beispiele für geeignete Amplifikationsstandards sind in SEQ ID NO. 8 und Abb. 5 gezeigt. Ein Beispiel für eine Fangsonde, die sich spezifisch zum Nachweis des in SEQ ID NO. 8 gezeigten Amplifikationsstandards eignet, ist das in SEQ ID NO. 9 dargestellte Oligonucleotid P5. Der Nachweis des in Abb. 5 gezeigten Standards kann z.B. durch das in SEQ ID NO. 9 dargestellte Oligonucleotid TE-CAT erfolgen.

Noch ein weiterer Gegenstand der vorliegenden Erfindung ist ein Reagenzienkit zum Nachweis von Telomerase-Aktivität, umfassend
(a) einen als Telomerasesubstrat geeigneten Primer,
(b) Nucleosidtriphosphate,
(c) Mittel zur Amplifikation des Telomerase-Extensionsprodukts,
(d) Markierungsgruppen
(e) Festphasenankergruppen und
(f) eine Festphase.

Die Markierungsgruppen sind vorzugsweise nicht-radioaktive Markierungsgruppen und können in Form von entsprechend markierten Nucleosidtriphosphaten oder/und markierten Primern vorliegen. Andererseits können die Markierungsgruppen auch auf einer oder mehreren Markierungssonden vorliegen, die unter Testbedingungen mit dem Amplifikationsprodukt hybridisieren.

Die Festphasenankergruppen sind vorzugsweise Biotin und die Festphase ist mit Streptavidin oder/und Avidin beschichtet. Besonders bevorzugt wird die Festphase aus Mikrotiterplatten, Mikroreaktionsgefäßen, Membranen, Mikrochips, Biocore-Systeme und gegebenenfalls magnetischen Mikrobeads ausgewählt.

Die Festphasenankergruppen können einerseits in Form von entsprechend modifizierten Nucleosidtriphosphaten bzw. auf einem Primer vorliegen. Andererseits können die Festphasenankergruppen auch auf einer Fangsonde vorliegen, die mit dem Amplifikationsprodukt hybridisiert.

Die Mittel zur Amplifikation des Telomerase-Extensionsprodukts umfassen vorzugsweise einen zweiten Primer und ein zur Amplifikation von Nucleinsäuren geeignetes Enzym, vorzugsweise eine thermostabile DNA-Polymerase.

In einer besonders bevorzugten Ausführungsform enthält der Reagenzienkit eine oder mehrere Markierungs- oder/und Fangsonden, die Markierungs- oder/und Festphasenankergruppen aufweisen und mit Amplifikationsprodukt hybridisieren. Die Markierungs- und Fangsonden werden vorzugsweise aus Oligonucleotiden und Nucleinsäureanaloga, insbesondere peptidischen Nucleinsäuren ausgewählt.

Sofern gewünscht, kann der Reagenzienkit auch Mittel zur weiteren Elongation eines Telomerase-Extensionsprodukt umfassen, z.B. ein Enzym wie Terminale Transferase oder DNA-Ligase.

Darüberhinaus kann der Reagenzienkit auch einen internen Standard zur Quantifizierung der Nachweisreaktion und entsrechende Mittel zum separaten Nachweis von Standard und Amplifikationsprodukt enthalten.

Noch ein weiterer Gegenstand der vorliegenden Erfindung ist die Verwendung eines Oligonucleotids aus dem 5'-Bereich der LTR-Sequenz von Retroviren als Telomerase-Substrat. Vorzugsweise stammt das Oligonucleotid aus dem 5'-Bereich der LTR-Sequenz von HIV und besonders bevorzugt weist das Oligonucleotid eine Länge von 10 - 50 Nucleotiden auf und umfaßt an seinem 3'-Ende (a) die in SEQ ID NO. 2 gezeigte Sequenz, (b) eine mindestens 80 % und insbesondere mindestens 90 % homologe Sequenz oder (c) mindestens die 10 letzten Nucleotide einer Sequenz gemäß (a) oder (b).

Weiterhin wird die vorliegende Erfindung durch die nachfolgenden Beispiele, Sequenzprotokolle und Abbildungen erläutert. Es zeigen:

| | |
|---|---|
| SEQ ID NO. 1 | einen als Telomerase-Substrat geeigneten Primer (P1); |
| SEQ ID NO. 2 | einen weiteren als Telomerase-Substrat geeigneten Primer (P-LTR); |
| SEQ ID NO. 3 | einen weiteren als Telomerase-Substrat geeigneten Primer (P1-Telo) ; |
| SEQ ID NO. 4 | einen Amplifikationsprimer (P2); |
| SEQ ID NO. 5 | einen zur Amplifikation geeigneten Ankerprimer (TE-ACT); |
| SEQ ID NO. 6 | einen zur Amplifikation geeigneten Oligo-dT-Primer (P3); |
| SEQ ID NO. 7 | eine Fangsonde (P4) ; |
| SEQ ID NO. 8 | einen Amplifikationsstandard für eine quantitative Bestimmung der Telomerase-Aktivität durch PCR; |
| SEQ ID NO. 9 | eine für einen standardisierten PCR-Ansatz verwendete Fangsonde (P5) ; |
| SEQ ID NO. 10 | eine weitere Fangsonde für einen standardisierten PCR-Ansatz (TE-CAT); |
| SEQ ID NO. 11 und 12 | zwei zur Herstellung eines Amplifikationsstandards verwendete Primer (P1-ST und TE-ACT-ST); SEQ ID NO. 13 einen zur Amplifikation geeigneten Ankerprimer (TE-3.2); |
| SEQ ID NO. 14 und 15 | zwei Kompetitor-Oligonucleotide. |

| | |
|---|---|
| Abb. 1 | eine schematische Darstellung von drei bevorzugten Ausführungsformen des erfindungsgemäßen Verfahrens; |
| Abb. 2 | das Ergebnis eines nicht-radioaktiven Nachweises von Telomerase im Mikrotiterplattenformat; |
| Abb. 3 | die Darstellung verschiedener erfindungsgemäßer Testführungen; |
| Abb. 4 | den Nachweis von Telomerase in Abhängigkeit von der eingesetzten Extraktmenge; |
| Abb. 5 | einen Amplifikationsstandard; |
| Abb. 6 | die Spezifität des Nachweises von Telomerase in Zellinien und Gewebeproben und |
| Abb. 7 | die Sensitivität des Telomerasenachweises in Zellinien und Gewebeproben. |

### Beispiele:

### 1. Reaktionsansatz zum Nachweis von Telomerase gemäß Version 1 des erfindungsgemäßen Verfahrens:

Es wurden 48 µl Telomerase/PCR-Puffer (20 mM Tris-HCl, pH 8, 3; 1,5 mM MgCl₂, 63 mM KCl, 0,05% Tween-20 (W/V); 1mM EGTA, 40 µM dNTP (N = A, G, C, U jeweils 10 µM), 300 nM Primer P1 oder P-LTR (SEQ ID NO. 1 oder 2) oder P1-Telo (SEQ ID NO. 3), 20 µg/ml T4g32-Protein, 0,1 mg/ml Rinderserumalbumin (RSA) (W/V), 2 U Taq DNA-Polymerase) in ein vorbeschichtetes PCR-Reaktionsgefäß vorgelegt. Dann wurden 2 µl einer Probe (z.B. S 100-Extrakt aus 10⁵ Zelläquivalenten) zugesetzt und 10 - 30 min bei 25°C inkubiert.

Das vorbeschichtete PCR-Reaktionsgefäß enthielt lyophilisiert an seinem Boden, mit einer Wachsschicht (z.B. AmliWax, Perkin Elmer) übergossen folgende Komponenten: 100 ng Primer P2 oder TE-ACT (SEQ ID NO. 4 oder 5; 223 nM in 50 µl), 30 ng DIG-dUTP (0,5 µM in 50 µl) und 370 ng biotinyliertes dUTP (7,5 µM in 50 µl).

Anschließend wurde eine PCR mit 25 Zyklen (30 s bei 94°C; 30 s bei 50°C, 90 s bei 72°C) durchgeführt. Beim Erhitzen auf 94°C schmolz die Wachsschicht und die lyophilisierten Reagenzien kam mit dem übrigen Reaktionsgemisch in Kontakt.

Nach der PCR wurde ein Aliquot des Ansatzes in eine mit Streptavidin-Thermo RSA-beschichtete Mikrotiterplatte überführt und dort 30 min bei 37°C inkubiert. Danach wurde dreimal mit jeweils 200 µl Waschpuffer (150 mM NaCl, 15 mM Na-Citrat, pH 7,0) gewaschen.

Dann erfolgte die Zugabe von Anti-DIG-Peroxidase-Konjugat (bei Verwendung einer anderen Markierungsgruppe ein entsprechendes anderes Peroxidase-Konjugat) und eine 60-minütige Inkubation bei 37°C. Dann wurde nochmals dreimal in jeweils 200 µl Waschpuffer gewaschen.

Zum Nachweis wurden 200 µl TMB-Substratreagenz (100 µg/ml 3,3',5,5'-Tetramethylbenzidin, 1 mM Citronensäure, 100 mM Na-Acetat, 0,01% H₂O₂) zugesetzt. Der Nachweis erfolgte in einem Mehrkanalphotometer bei einer Wellenlänge von 450 nm.

### 2. Reaktionsansatz zum Nachweis von Telomerase gemäß Version 2 des erfindungsgemäßen Verfahrens:

Die Reaktion wurde bis zum Telomerase-Extensionsschritt wie unter Punkt 1 beschrieben durchgeführt. Dann wurden 2,5 U/µl Terminale Transferase in 200 mM K-Cacodylat, 5mM CoCl₂, zugegeben und 60 min bei 37°C inkubiert.

Anschließend wurde eine PCR wie unter Punkt 1 beschrieben durchgeführt. Anstelle von Primer P2 wurde der Primer P3 mit der in SEQ ID NO. 6 angegebenen Sequenz verwendet.

Der Nachweis der Amplifikationsprodukte erfolgte wie unter Punkt 1 beschrieben.

Alternativ zu den oben beschriebenen Verfahren wurde die Immobilisierung der Amplifikationsprodukte auch durch Verwendung von biotinylierten Telomerase/PCR-Primer (P1-Bio oder P1.TeloBio) anstelle des Einbaus von biotinyliertem dUTP durchgeführt.

### 3. Reaktionsansatz zum Nachweis von Telomerase gemäß Version 3 des erfindungsgemäßen Verfahrens:

Die Telomerase-Extension erfolgte wie unter Punkt 1 beschrieben. Das vorbeschichtete PCR-Reaktionsgefäß enthielt dieselben Komponenten wie unter Punkt 1 beschrieben, aber kein biotinyliertes Nucleosidtriphosphat.

Die Durchführung der PCR erfolgte wie unter Punkt 1 beschrieben. Nach der PCR wurden 10 µl des Ansatzes in 40 µl Denaturierungspuffer (125 mM NaOH, 0,2 mM EDTA) gegeben und 10 min bei Raumtemperatur inkubiert.

Dann wurden 450 µl Hybridisierungspuffer (62,5 mM Na-Phosphat, pH 6,5, 630 mM NaCl, 0,0625% RSA (W/V) und 1 µM biotinylierte Fangsonde, z.B. Oligonucleotid P1-Bio oder P4) zugegeben und 200 µl des Ansatzes in eine mit SA-Thermo RSA-beschichtete Mikrotiterplatte überführt. Dann wurde 60 min bei 37°C inkubiert und anschließend dreimal mit jeweils 200 µl Waschpuffer gewaschen.

Der Nachweis der Amplifikationsprodukte erfolgte wie unter Punkt 1 beschrieben.

Alle Varianten des erfindungsgemäßen Verfahrens wurden alternativ auch mit einer sequentiellen Testführung ohne Kompartimentierung durch Wachs durchgeführt. Die Gesamtdauer aller Testformate betrug maximal 6 h.

### 4. Reaktionsansatz zum Nachweis von Telomerase gemäß Version 4 des erfindungsgemäßen Verfahrens:

Der Telomerase-Extensionsschritt wurde wie unter Punkt 1 durchgeführt. Als Telomerase-Substrate wurden sowohl unbiotinylierte als auch biotinylierte Primer verwendet. Im Gegensatz zu Version 1 wurde kein vorbeschichtetes Reaktionsgefäß verwendet.

Die nachfolgende PCR wurde, wie unter Punkt 1 beschrieben, aber in Abwesenheit von markierten Nucleotiden durchgeführt.

Nach der Amplifikation erfolgte eine Denaturierung wie unter Punkt 3 beschrieben. Bei Verwendung eines biotinylierten Primers wurde eine DIG-markierte Markierungssonde und bei Verwendung eines unbiotinylierten Primers eine DIG-markierte Markierungssonde und eine biotinylierte Fangsonde zugegeben.

Der Nachweis der Amplifikationsprodukte erfolgte wie unter Punkt 1 beschrieben.

### 5. Standardisierter Reaktionsansatz:

Mit Hilfe der Primer P1-ST (SEQ ID NO. 11) und TE-ACT-ST (SEQ ID NO. 12) wurde ein Sequenzbereich aus der kodierenden Region des bakteriellen Enzyms Chloramphilicol-Acetyl-Transferase amplifiziert. Das resultierende 202 Bp lange PCR-Fragment (Abb. 5) enthielt Sequenzen aus dem Chloramphenicol-Acetyltransferase-Gen, die von den Sequenzen der beiden Telomerase Primer P1 (SEQ ID NO. 1) und TE-ACT (SEQ ID NO.5) flankiert werden.

Beim Telomerase-Extensionsschritt wurde eine definierte Menge dieses PCR-Produkts (1 - 20 attomol) zugegeben, das - bei Verwendung der entsprechenden Primer - ebenso wie die Telomerase-Extensionsprodukte amplifiziert wurde.

Der Nachweis und die Quantifizierung des Standards erfolgte über Hybridisierung eines DIG-markierten, CAT-spezifischen Primers TE-CAT (SEQ ID NO. 10), wenn während der Amplifikation biotinylierter Primer eingesetzt wurde, oder durch Hybridisierung mit einer biotinylierten, CAT-spezifischen Fangsonde, wenn der Einbau von Markierungsgruppen während der Amplifikation erfolgte.

### 6. Reaktionsansatz PCR-ELISA

Der Telomerase-Extensionsschritt und die Amplifikation wurden in einem einzigen, nicht-vorbeschichteten Reaktionsgefäß durchgeführt.

Hierzu wurden der Probe (1 - 3 µl Zellextrakt entsprechend 1 x 10³ bis 3 x 10³ Zelläquivalenten oder 1 - 50 µg Gesamtprotein) bzw. einer positiven Kontrolle (Extrakt aus Zellen mit bekannter Telomerase-Aktivität) oder einer negativen Kontrolle (RNase- oder hitzebehandelter Zellextrakt) ein Reaktionsgemisch zugegeben, welches einen biotinylierten Telomerase-Primer (z.B. P1, t-LTR oder P1-Telo) einen Ankerprimer (TE-ACT oder TE-3.2), unmarkierte dNTP und thermostabile DNA-Polymerase enthielt.

Das Reaktionsgemisch wurde zur Durchführung einer kombinierten Primerelongation/Amplifikation in einen Thermocycler gegeben. Dort wurde zunächst eine Primerelongation für 10 - 30 min bei 25°C durchgeführt. Dann wurde 5 min zur Inaktivierung der Telomerase auf 94°C erhitzt. Anschließend wurde wie unter Punkt 1 beschrieben eine PCR mit 30 Zyklen durchgeführt, wonach ein zehnminütiges Erhitzen auf 72°C folgte.

Ein Aliquot des Amplifikationsansatzes wurde dann denaturiert und in Anwesenheit unmarkierter Kompetitor-Oligonucleotide mit einer DIG-markierten Fangsonde hybridisiert. Das resultierende Produkt wurde mittels der Biotingruppen an eine Streptavidinbeschichtete Mikrotiterplatte immobilisiert.

Der Nachweis der immobilisierten Amplifikationsprodukte erfolgte wie unter Punkt 1 beschrieben. Alternativ wurde der Reaktionsansatz gelelektrophoretisch aufgetrennt (z.B. nichtdenaturierendes Polyacrylamidgel), und die Banden auf eine Membran transferiert und dort sichtbar gemacht.

### 7. Ergebnisse:

In Abbildung 1 sind die unter den Punkten 1 - 3 beschriebenen Testformate exemplarisch dargestellt.

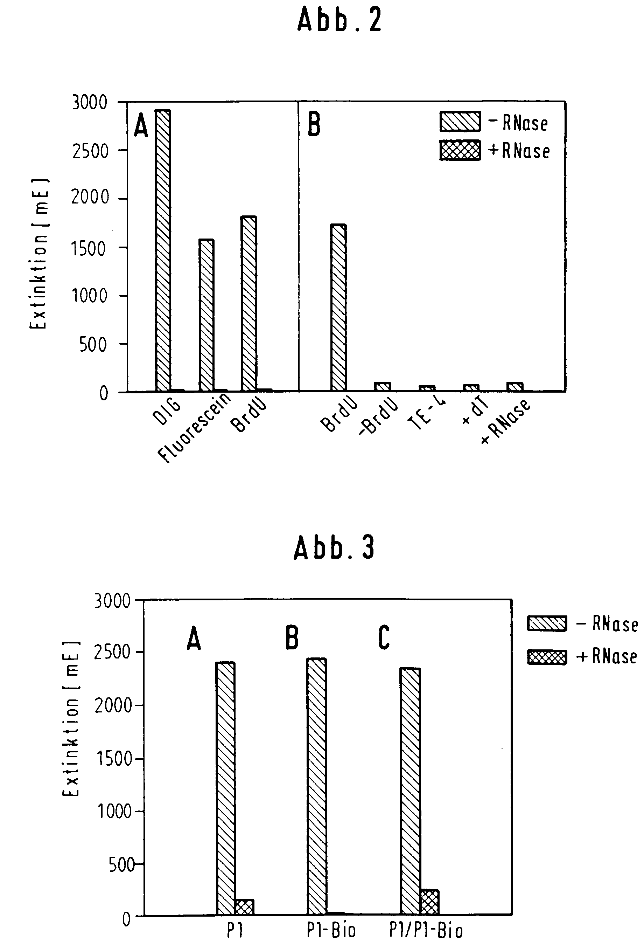

Abbildung 2 zeigt den nicht-radioaktiven Nachweis von Telomerase nach Immobilisierung auf Mikrotiterplatten. Pro 50 µl Ansatz (Primer: P1-Bio) wurden 1 x 10⁵ Zelläquivalente (HeLa-Extrakt) eingesetzt. A: Die Einführung der Markierungsgruppen erfolgte während des PCR-Schritts mit den jeweils angegebenen Nucleotiden (DIG-dUTP, Fluorescein-dUTP, Br-dUTP), die Immobilisierung erfolgte in einer Streptavidin-beschichteten Mikrotiterplatte und der Nachweis mit den entsprechenden Antikörper-Konjugaten. B: Spezifität der Reaktion: Bei Zusatz von RNase, durch den die Telomerase-Aktivität inhibiert wird, konnten keine Amplifikationsprodukte nachgewiesen werden.

Abbildung 3 zeigt das Ergebnis bei verschiedenen Testformaten. A: das Ergebnis eines Doppelmarkierungsexperiments (DIG-dUTP/Bio-dUTP) gemäß Version 1; B: Einfachmarkierungsexperiment (DIG-dUTP) unter Verwendung eines biotinylierten Primers P1 (P1-Bio) gemäß Version 1; C: Einfachmarkierungsexperiment (DIG-dUTP) unter Verwendung des Primers P1 als Telomerase-Substrat und des Primers P1-Bio als Fangsonde gemäß Version 3.

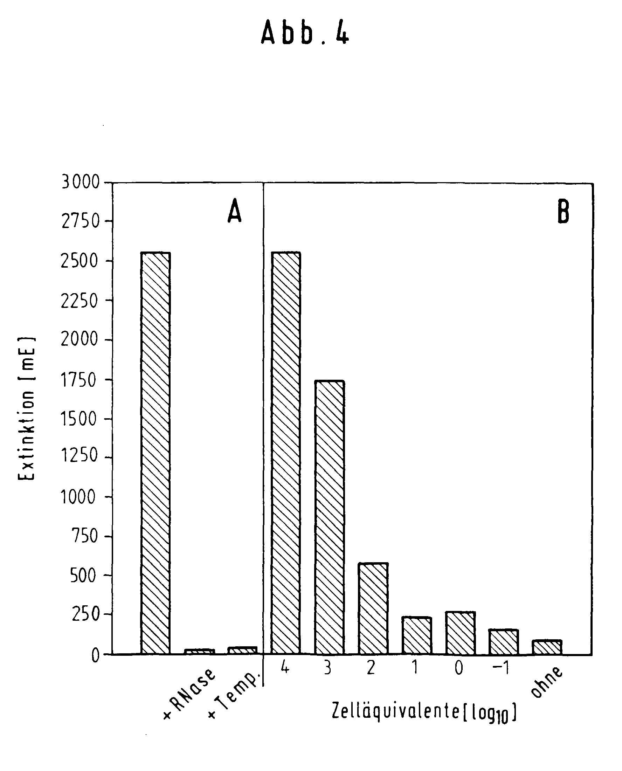

Abbildung 4 zeigt den Nachweis von Telomerase in Abhängigkeit von eingesetzten Extraktmenge. Es wurde ein Einfachmarkierungsexperiment (DIG-dUTP) unter Verwendung des biotinylierten Primers P1-Bio gemäß Version 1 durchgeführt. A: Spezifität des Tests. Nach RNase- bzw. Temperaturbehandlung konnte kein Amplifikationsprodukt mehr nachgewiesen werden. B: Sensitivität des Tests: Ausgehend von 1 x 10⁴ Zelläquivalenten wurde der eingesetzte Extrakt (-/+ RNase-Behandlung) log₁₀ titriert. 5% des PCR-Ansatzes wurden anschließend zum Nachweis auf einer Streptavidin-beschichteten Mikrotiterplatte eingesetzt. Aus Abbildung 4 ist ersichtlich, daß 1 - 10 Zelläquivalente noch deutlich über dem Hintergrund (ohne) liegende Signale ergeben.

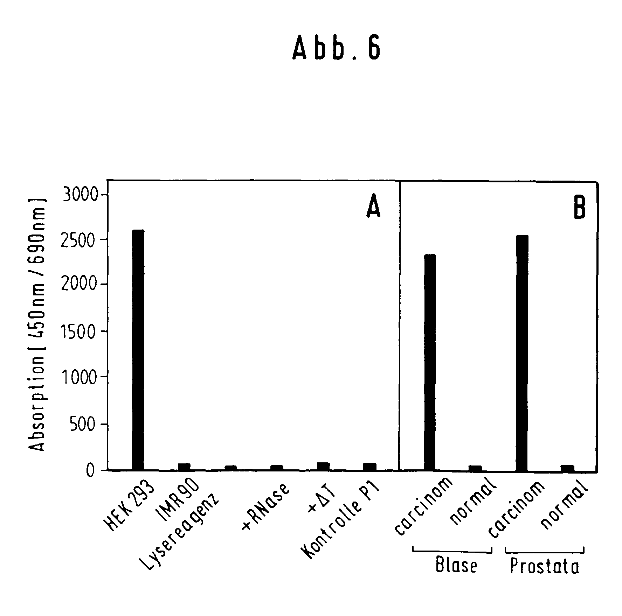

Abbildung 6 zeigt den spezifischen Nachweis von Telomeraseaktivität in Zellinien und Gewebeproben. A: Die humane Telomerasepositive Embryo-Nierenzellinie 293 und humane Telomerasenegative Lungenfibroblastenzellinie EMR90 wurden durch einen erfindungsgemäßen Telomerase PCR-ELISA (Punkt 6) analysiert. Als negative Kontrollen wurden ein Lysereagenz ohne Extrakt (Lysereagenz), mit RNase behandelte 293-Zellen (+RNase) oder hitzebehandelte 293-Zellen (+ΔT) verwendet. Alle Kontrollen ergaben negative Ergebnisse. Die Kontrolle P1 ist ein synthetisches Oligonucleotid, das von der Telomerase nicht als Substrat akzeptiert wird. Die Tests wurden wie unter Punkt 6 beschrieben mit einer Extraktmenge entsprechend jeweils 1 x 10³ Zelläquivalenten duchgeführt. B: Es wurde die Telomeraseaktivität in durch Biopsien erhaltenem Normalgewebe und primärem Tumorgewebe analysiert. Dabei wurden ein Prostatakarzinom und ein Blasenkarzinom jeweils mit normalem Prostata- und Blasengewebe (normal) verglichen. Die Tests wurden wie in Abschnitt (A) beschrieben unter Verwendung von 20 µg Gesamtprotein durchgeführt.

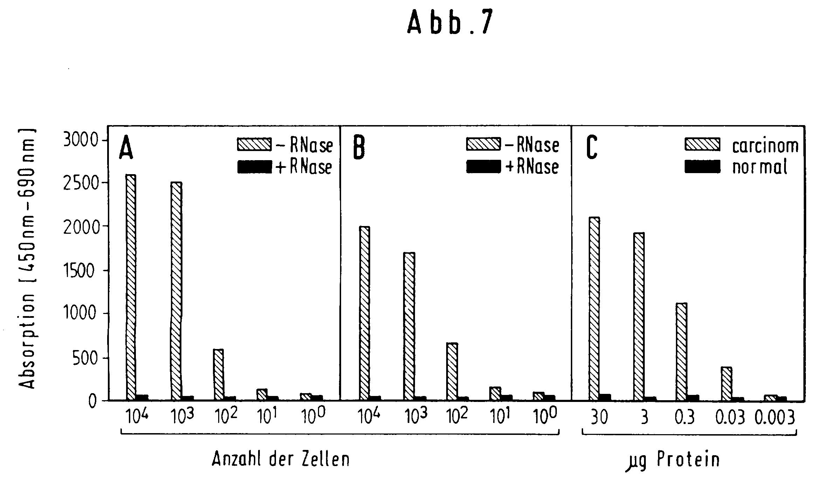

Abbildung 7 zeigt die Sensitivität des erfindungsgemäßen Telomerase PCR-ELISA. A: Ein Extrakt von Telomerase-positiven 293-Zellen wurde seriell mit Lysereagenz verdünnt. Die jeweils angegebenen Zelläquivalente wurden wie unter Punkt 6 beschrieben analysiert. Die Ergebnisse sind für RNase-behandelte Extrakte (+RNase) oder nicht mit RNase behandelte Extrakte (-RNase) angegeben. B: 293-Zellen wurden seriell in Kulturmedium vor der Lyse verdünnt und dann mit Lysereagenz wie zuvor beschrieben behandelt. Die angegebene Anzahl von Zellen wurde im Telomerase PCR-ELISA analysiert. Die Tests wurden wie zuvor beschrieben durchgeführt. Es sind Ergebnisse für mit RNase behandelte Proben (+RNase) sowie für Proben ohne RNase-Behandlung (-RNase) angegeben. C: Es wurde die Telomeraseaktivität in seriell verdünnten Extrakten gemessen, die aus Blasenkarzinomtumorgewebe und normalem Blasengewebe erhalten wurden. Die angegebenen Mengen an Gewebematerial wurden wie zuvor beschrieben getestet.

## Patentansprüche

1. Verfahren zum Nachweis von Telomerase-Aktivität,
**dadurch gekennzeichnet,**
daß man
(a) eine zu testende Probe bereitstellt,
(b) einen als Telomerasesubstrat geeigneten ersten Primer und Nucleosidtriphosphate zugibt und das Reaktionsgemisch unter Bedingungen inkubiert, bei denen eine Primerextension durch die Telomerase stattfinden kann,
(c) eine Amplifikation des durch die Telomerase erzeugten Extensionsprodukts durchführt,
(d) das in Schritt (c) erzeugte Amplifikationsprodukt an einer Festphase immobilisiert und
(e) das immobilisierte Amplifikationsprodukt qualitativ oder/und quantitativ nachweist.

2. Verfahren nach Anspruch 1,
**dadurch gekennzeichnet,**
daß der Nachweis des Amplifikationsprodukts über nicht-radioaktive
Markierungsgruppen erfolgt.

3. Verfahren nach Anspruch 1 oder 2,
**dadurch gekennzeichnet,**
daß die nicht-radioaktiven Markierungsgruppen ausgewählt werden aus immunologisch reaktiven Gruppen, lumineszierenden Gruppen und fluoreszierenden Gruppen.

4. Verfahren nach einem der Ansprüche 1 - 3,
**dadurch gekennzeichnet,**
daß eine direkte Markierung des Amplifikationsprodukts durch Einbau von Markierungsgruppen erfolgt.

5. Verfahren nach einem der Ansprüche 1 - 3,
**dadurch gekennzeichnet,**
daßeine indirekte Markierung des Amplifikationsprodukts durch Hybridisierung mit einer Markierungssonde erfolgt.

6. Verfahren nach einem der Ansprüche 1 - 5,
**dadurch gekennzeichnet,**
daß die Festphase ausgewählt wird aus Mikrotiterplatten, Mikroreaktionsgefäßen, Membranen, Mikrochips, Biocore-Systemen und gegebenenfalls magnetischen Mikrobeads.

7. Verfahren nach einem der Ansprüche 1 - 6,
**dadurch gekennzeichnet,**
daß die Amplifikation durch PCR oder LCR erfolgt.

8. Verfahren nach einem der Ansprüche 1 - 7,
**dadurch gekennzeichnet,**
daß man einen ersten Primer verwendet, der frei von telomeren Repeatsequenzen ist.

9. Verfahren nach Anspruch 8,
**dadurch gekennzeichnet,**
daß man einen ersten Primer verwendet, der aus dem 5'-Bereich einer retroviralen LTR-Sequenz stammt.

10. Verfahren nach Anspruch 9,
**dadurch gekennzeichnet,**
daß man einen ersten Primer aus dem 5'-Bereich der LTR-Sequenz von HIV verwendet.

11. Verfahren nach einem der Ansprüche 1 - 10,
**dadurch gekennzeichnet,**
daß man einen ersten Primer verwendet, der telomere Repeatsequenzen enthält.

12. Verfahren nach einem der Ansprüche 7 - 11,
**dadurch gekennzeichnet,**
daß man bei der Amplifikation einen zweiten Primer verwendet, der an seinem 5'-Ende einen nicht zur telomeren Repeatsequenz komplementären Bereich enthält.

13. Verfahren nach einem der Ansprüche 1 - 12,
**dadurch gekennzeichnet,**
daß man nach dem Extensionsschritt (b) eine weitere Template-unabhängige Elongation des Extensionsprodukts durchführt.

14. Verfahren nach einem der Ansprüche 1 - 13,
**dadurch gekennzeichnet,**
daß der Extensionsschritt (b) so durchgeführt wird, daß nur unmarkierte Nucleosidtriphosphate an den ersten Primer angefügt werden können.

15. Verfahren nach einem der Ansprüche 1 - 14,
**dadurch gekennzeichnet,**
daß die Immobilisierung an der Festphase über Ankergruppen erfolgt, die in die Amplifikationsprodukte durch Verwendung von Ankergruppen enthaltenden Primern oder/und durch Verwendung von Ankergruppen enthaltenden Nucleosidtriphosphaten insbesondere während des Amplifikationsschritts (c) eingeführt werden.

16. Verfahren nach einem der Ansprüche 1 - 15,
**dadurch gekennzeichnet,**
daß die Immobilisierung an der Festphase über Ankergruppen erfolgt, die in einer mit dem Amplifikationsprodukt hybridisierenden Fangsonde enthalten sind.

17. Verfahren nach einem der Ansprüche 1 - 16,
**dadurch gekennzeichnet,**
daß man bei der Amplifikation einen internen Standard zusetzt, der einen quantitativen Nachweis der Amplifikationsprodukte ermöglicht.

18. Verfahren nach einem der Ansprüche 1 - 17,
**dadurch gekennzeichnet,**
daß man die Reaktion als Ein-Topf-Reaktion durchführt.

19. Verfahren nach einem der Ansprüche 1 - 18,
**dadurch gekennzeichnet,**
daß man die Immobilisierung oder/und den Nachweis des Amplifikationsprodukts unter solchen Bedingungen durchführt, die eine spezifische Detektion des Telomeraseextensionsprodukts ohne Abtrennung von Amplifikationsnebenprodukten erlauben.

20. Reagenzienkit zum Nachweis von Telomerase-Aktivität, umfassend
(a) einen als Telomerasesubstrat geeigneten Primer,
(b) Nucleosidtriphosphate,
(c) Mittel zur Amplifikation des Telomerase-Extensionsprodukts,
(d) Markierungsgruppen
(e) Festphasenankergruppen und
(f) eine Festphase.

21. Reagenzienkit nach Anspruch 20,
**dadurch gekennzeichnet,**
daß die Festphase aus Mikrotiterplatten, Mikroreaktionsgefäßen, Membranen, Mikrochips, Biocore-Systemen und gegebenenfalls magnetischen Mikrobeads ausgewählt ist.

22. Verwendung eines Oligonucleotids aus dem 5'-Bereich der LTR-2 Sequenz von Retroviren als Primer in einem Verfahren nach einem der Ansprüche 1 bis 19 als Telomerase-Substrat.

23. Verwendung eines Oligonucleotids mit einer Länge von 10 - 50 Nucleotiden, das an seinem 3'-Ende (a) die in SEQ ID NO. 2 gezeigte Sequenz, (b) eine dazu mindestens 80 % homologe Sequenz oder (c) mindestens die 10 letzten Nucleotide einer Sequenz gemäß (a) oder (b) umfaßt, als Primer in einem Verfahren nach einem der Ansprüche 1 bis 19.

24. Verwendung eines Oligonucleotids oder eines Nucleinsäureanalogons mit einer zur telomeren Repeatsequenz komplementären Sequenz und 5'-seitig davon einer Extensionssequenz, die mindestens 4 und insbesondere 4 - 10 Nucleotide lang ist, als Primer in einem Verfahren nach Anspruch 1 zum Nachweis von Telomerase-Aktivität.

## Claims

1. Method for the detection of telomerase activity, **wherein**
(a) a sample to be tested is prepared,
(b) a first primer suitable as a telomerase substrate and nucleoside triphosphates are added and the reaction mixture is incubated under conditions under which a primer extension by the telomerase can take place,
(c) an amplification of the extension product produced by the telomerase is carried out,
(d) the amplification product produced in step (c) is immobilized on a solid phase and
(e) the immobilized amplification product is detected qualitatively or/and quantitatively.

2. Method as claimed in claim 1,
**wherein**
the amplification product is detected via non-radioactive labelling groups.

3. Method as claimed in claim 1 or 2,
**wherein**
the non-radioactive labelling groups are selected from immunologically reactive groups, luminescent groups and fluorescent groups.

4. Method as claimed in one of the claims 1 - 3,
**wherein**
the amplification product is directly labelled by incorporation of labelling groups.

5. Method as claimed in one of the claims 1 - 3,
**wherein**
the amplification product is indirectly labelled by hybridization with a labelling probe.

6. Method as claimed in one of the claims 1 - 5,
**wherein**
the solid phase is selected from microtitre plates, microreaction vessels, membranes, microchips, biocore systems and optionally magnetic microbeads.

7. Method as claimed in one of the claims 1 - 6,
**wherein**
the amplification is achieved by PCR or LCR.

8. Method as claimed in one of the claims 1 - 7,
**wherein**
a first primer is used which is free of telomere repeat sequences.

9. Method as claimed in claim 8,
**wherein**
a first primer is used which is derived from the 5' region of a retroviral LTR sequence.

10. Method as claimed in claim 9,
**wherein**
a first primer is used from the 5' region of the LTR sequence of HIV.

11. Method as claimed in one of the claims 1 - 10,
**wherein**
a first primer is used which contains telomere repeat sequences.

12. Method as claimed in one of the claims 7 - 11,
**wherein**
a second primer is used in the amplification which contains at its 5' end a region which is not complementary to the telomere repeat sequence.

13. Method as claimed in one of the claims 1 - 12,
**wherein**
a further template-independent elongation of the extension product is carried out after the extension step (b).

14. Method as claimed in one of the claims 1 - 13,
**wherein**
the extension step (b) is carried out in such a way that only unlabelled nucleoside triphosphates can be attached to the first primer.

15. Method as claimed in one of the claims 1 - 14,
**wherein**
the immobilization to the solid phase is achieved via anchor groups which are introduced into the amplification products especially during the amplification step (c) by using primers containing anchor groups or/and by using nucleoside triphosphates containing anchor groups.

16. Method as claimed in one of the claims 1 - 15,
**wherein**
the immobilization to the solid phase is achieved via anchor groups which are contained in a capture probe that hybridizes with the amplification product.

17. Method as claimed in one of the claims 1 - 16,
**wherein**
an internal standard is added in the amplification which enables a quantitative detection of the amplification products.

18. Method as claimed in one of the claims 1 - 17,
**wherein**
the reaction is carried out as a one-pot reaction.

19. Method as claimed in one of the claims 1 - 18,
**wherein**
the immobilization or/and the detection of the amplification product is carried out under conditions that enable a specific detection of the telomerase extension product without separating amplification byproducts.

20. Reagent kit for the detection of telomerase activity comprising
(a) a primer suitable as a telomerase substrate,
(b) nucleoside triphosphates,
(c) agents for the amplification of the telomerase extension product,
(d) labelling groups
(e) solid phase anchor groups and
(f) a solid phase.

21. Reagent kit as claimed in claim 20,
**wherein**
the solid phase is selected from microtitre plates, microreaction vessels, membranes, microchips, Biocore systems and optionally magnetic microbeads.

22. Use of an oligonucleotide from the 5' region of the LTR-2 sequence of retroviruses as the telomerase substrate as claimed in one of the claims 1 to 19.

23. Use of an oligonucleotide with a length of 10 - 50 nucleotides which comprises at its 3' end (a) the sequence shown in SEQ ID NO. 2, (b) a sequence which is at least 80 % homologous thereto or (c) at least the last 10 nucleotides of a sequence according to (a) or (b) as primers in a method as claimed in one of the claims 1 to 19.

24. Use of an oligonucleotide or a nucleic acid analogue containing a sequence which is complementary to the telomere repeat sequence and an extension sequence at the 5' side thereof which has a length of at least 4 and in particular 4 - 10 nucleotides as a primer in a method as claimed in claim 1 for the detection of telomerase activity.

## Revendications

1. Procédé pour la détection de l'activité d'une télomérase,
caractérisé en ce que,
l'on
(a) prépare une éprouvette à tester,
(b) on ajoute un premier amorceur approprié comme substrat de télomérase et un triphosphate de nucléoside et l'on met en incubation le mélange réactionnel dans des conditions où un allongement d'amorceur peut s'effectuer par la télomérase,
(c) on effectue une amplification du produit d'allongement généré par la télomérase,
(d) on immobilise le produit d'amplification généré à l'étape (c) sur une phase solide et
(e) on détermine qualitativement et/ou quantitativement le produit d'amplification immobilisé.

2. Procédé selon la revendication 1,
caractérisé en ce que,
la détection du produit d'amplification s'effectue par des groupes de marquage non radioactifs.

3. Procédé selon la revendication 1 ou 2,
caractérisé en ce que,
les groupes de marquage non radioactifs sont choisis à partir de groupes immunologiquement réactifs, de groupes luminescents et de groupes fluorescents.

4. Procédé selon l'une des revendications 1-3,
caractérisé en ce
qu'il est produit un marquage directe du produit d'amplification par incorporation de groupes de marquage.

5. Procédé selon l'une des revendications 1-3,
caractérisé en ce que,
l'on procède à un marquage indirecte du produit d'amplification par hybridation avec une sonde de marquage.

6. Procédé selon l'une des revendications 1-5,
caractérisé en ce que,
la phase solide est choisie à partir de plaques de microtitrage, de récipients de microréaction, de membranes, de micropuces, de systèmes biocore et éventuellement de microperles magnétiques.

7. Procédé selon l'une des revendications 1-6,
caractérisé en ce que,
l'amplification s'effectue par PCR ou LCR.

8. Procédé selon l'une des revendications 1-7,
caractérisé en ce que,
l'on utilise un premier amorceur qui est exempt de séquences répétitives de télomère.

9. Procédé selon la revendication 8,
caractérisé en ce que,
l'on utilise un premier amorceur qui provient de la zone 5' d'une séquence LTR rétrovirale.

10. Procédé selon la revendication 9,
caractérisé en ce que,
l'on utilise un premier amorceur à partir de la zone 5' de la séquence LTR de HIV.

11. Procédé selon l'une des revendications 1-10,
caractérisé en ce que,
l'on utilise un premier amorceur qui contient des séquences répétitives de télomère.

12. Procédé selon l'une des revendications 7-11,
caractérisé en ce que,
l'on utilise lors d'une amplification un deuxième amorceur qui sur son extrémité 5' contient une zone non complémentaire pour la séquence répétitive télomère.

13. Procédé selon l'une des revendications 1-12,
caractérisé en ce que,
l'on effectue après l'étape d'allongement (b) une autre élongation indépendante de la matrice du produit d'allongement.

14. Procédé selon l'une des revendications 1-13,
caractérisé en ce que,
l'étape d'allongement (b) est conduite de telle sorte que seul le triphosphate de nucléoside non marqué peut être annexé au premier amorceur.

15. Procédé selon l'une des revendications 1-14,
caractérisé en ce que,
l'immobilisation sur la phase solide s'effectue par des groupes d'ancrage qui sont introduits dans les produits d'amplification par utilisation d'amorceurs contenant des groupes d'ancrage ou/et par utilisation de triphosphates de nucléoside contenant des groupes d'ancrage, notamment pendant l'étape d'amplification (c).

16. Procédé selon l'une des revendications 1-15,
caractérisé en ce que,
l'immobilisation sur la phase solide s'effectue par des groupes d'ancrage qui sont contenus dans une sonde de capture s'hybridant avec le produit d'amplification.

17. Procédé selon l'une des revendications 1-16,
caractérisé lors de l'amplification on ajoute un étalon interne qui permet une détection quantitative des produits d'amplification.

18. Procédé selon l'une des revendications 1-17,
caractérisé en ce que l'on effectue la réaction en récipient unique.

19. Procédé selon l'une des revendications 1-18,
caractérisé en ce que,
l'on effectue l'immobilisation ou/et la détection du produit d'amplification dans les conditions telles permettant la détection spécifique du produit d'allongement de télomérase sans séparation des sous-produits d'amplification.

20. Kit de réactif pour la détection de l'activité de télomérase, comprenant :
(a) un amorceur convenant comme substrat de télomérase,
(b) triphosphate de nucléoside,
(c) moyen pour l'amplification du produit d'extension-télomérase,
(d) groupes de marquage
(e) groupes d'ancrage phase solide et
(f) une phase solide

21. Kit de réactif selon la revendication 20,
caractérisé en ce que,
la phase solide est choisie à partir de plaques de microtitrage, de récipients de microréaction, de membranes de micropuces, de systèmes biocore et éventuellement de microperles magnétiques.

22. Utilisation d'un oligonucléotide à partir de la zone 5' de la séquence LTR-2 des rétrovirus en tant qu'amorceur dans un procédé selon l'une des revendications 1 à 19 comme substrat de télomérase.

23. Utilisation d'un oligonucléotide ayant une longueur de 10-50 nucléotides, qui sur son extrémité 3' (a) comprend la séquence montrée dans SEQ ID NO.2, (b) une séquence homologue à celle-ci à au moins 80% ou (c) au moins les dix derniers nucléotides d'une séquence selon (a) ou (b), en tant qu'amorceur dans un procédé selon l'une des revendications 1 à 19.

24. Utilisation d'un nucléotide ou d'un analogon de l'acide nucléique avec une séquence complémentaire à la séquence répétitive télomère et sur le côté 5' de celle-ci, une séquence d'allongement qui a une longueur d'au moins 4 et en particulier 4-10 nucléotides en tant qu'amorceur dans un procédé selon la revendication 1 pour la détection de l'activité de la télomérase.
